# EUROPEAN PATENT SPECIFICATION

(11) **EP 2 358 440 B1**
(45) Date of publication and mention of the grant of the patent: **30.03.2016**
(21) Application number: 09760760.0
(22) Date of filing: 25.11.2009
(51) Int. Cl.: A61Q 5/04, A61K 8/35, A61K 8/22

(54) **PROCESS FOR PERMANENT SHAPING OF HUMAN HAIR**
ZUSAMMENSETZUNG ZUR DAUERHAFTEN VERFORMUNG DES MENSCHLICHEN HAARES
COMPOSITION DE MISE EN FORME PERMANENTE DES CHEVEUX HUMAINS

(30) Priority: 26.11.2008 EP 08020519
(43) Date of publication of application: 24.08.2011
(73) Proprietor: Kao Germany GmbH, 64297 Darmstadt (DE)
(72) Inventor: WOOD, Jonathan, 69469 Weinheim (DE); HULLMANN, Alexandra, 63329 Egelsbach (DE); SCHNEIDER, Jörg, 64347 Griesheim (DE)
(74) Representative: Grit, Mustafa
(86) International application number: PCT/EP2009/008377
(87) International publication number: WO 2010/060602

(56) References cited:
- EP-A- 1 269 976
- EP-A- 1 797 861
- EP-A- 2 020 255
- DE-A1- 10 106 745

## Description

The present invention concerns a process for permanent shaping of human hair used both for the permanent waving of human hair with an excellent waving effect as well as for the straightening of curled hair.

It is generally known that permanent waving is carried out according to a two step process. In the first step, the reductive splitting of the cysteine disulfide bonds is achieved by a reducing agent, and in the second step, neutralization is carried out by application of an oxidizing agent, whereby the cysteine disulfide bonds are restored in the new shape.

The reducing agent still most frequently used today is thioglycolic acid, also in form of the salts thereof, in particular its ammonium salt, although numerous other thio compounds have been proposed for this purpose, which, however, mostly did not succeed.

The compositions containing thioglycolates are customarily applied at a pH-value between 7 and 10, in particular 8.5 and 9.5.

It is also well known in the art that use of an intermediate treatment is advantageous in order to realize more efficient perming and reduce hair damage. These compositions usually comprise only salts at higher concentration in order to de-swell hair which occurs usually when hair is treated with strong alkaline compositions especially in the presence of a reducing agent. Although the prior art has quite developed, there is still need for further improvements, especially in realizing lower hair damage without loss of perming and/or straightening efficiency.

The present invention starts from the task of providing a process for the permanent shaping of human hair wherein an intermediate treatment composition is used with excellent waving and straightening performance. Hair waved or straightened according to the process of the present invention looks attractive and feels natural upon touching by hand.

Accordingly, the first object of the present invention is a process for permanent shaping hair wherein a composition comprising at least one reducing agent is applied onto hair and after processing of 1 to 30 min at a temperature of 20 to 45°C rinsed off from hair and an intermediate composition comprising at least one ubiquinone of the formula 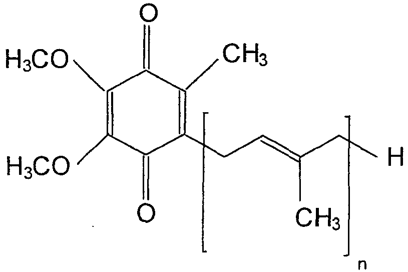 where n is a number between 1 and 10, and at least one salt and at least one oxidizing agent is applied onto hair and after processing of 10 seconds to 10 min and without rinsing off a composition comprising at least one oxidizing agent is applied and rinsed off from hair after a processing time of 1 to 30 min and at a temperature of 20 to 45°C.

In a further preferred form of the invention the hair is washed and more preferably shampooed prior to application of the reducing composition.

In case that the aim of using the process is perming (curling), prior to application of the reducing agent or during the application of the reducing agent, even after the application of the reducing agent, hair is put on the curlers and the curlers are taken out after application of the intermediate treatment composition or after application of the oxidizing composition or after processing of the oxidizing composition. The selection of the timing when the curlers are put and taken off from hair is very much dependent on the curling efficiency aimed. For stronger curls it is preferred that the curlers are put on the hair before application of the reducing agent. Putting curlers onto hair during or after the application of the reducing agent produces relatively weaker curls. For stronger curls it is preferred that the curlers are taken off at the end of the processing time of oxidizing agent, and for relatively weaker, slight curling effect, curlers are taken off right after application of the intermediate treatment.

The reducing composition used according to the inventive process in the first step comprises at least one reducing compound. Preferred are thioglycolic acid and thiolactic acid as well as the salts thereof, in particular the ammonium and ethanolamine salts. Further useful thio compounds are in particular cysteine or the hydrochloride thereof, homocysteine, cysteamine, N-acetyl cysteine, thioglycerol, ethanediol monothioglycolate, 1,2-propyleneglycol monothioglycolate (see also WO-A 93/1791), 1,3-propanediol monothioglycolate or the isomer mixture resulting therefrom, 1,3-butanediol and 1,4-butanediol monothioglycolate and the isomer mixtures therefrom, polyethylene glycol, such as di-, tri- and tetraethyleneglycol monothioglycolates, glycerol monothiolactate and further thio acids and the esters thereof, as well as mixtures thereof.

The use of inorganic reducing sulfur compounds such as sodium hydrogen sulfite is basically also possible.

The total reduction agent content in the compositions according to the invention customarily amounts from 0.5 % to 15 %, preferably 1 to 15%, more preferably 1 to 12.5% and most preferably 1 to 12.5% by weight, calculated to total composition as free thioglycolic acid as reference substance.

The composition comprising reducing agents can, if necessary, comprise alkalizing agents. Their quantity is dependent on the reducing agent and the desired pH-value of the composition. Reducing agent compositions preferably contain about 0.1 % to about 5%, in particular about 0.5% to about 2.5% by weight thereof, calculated to the total composition. Alkalizing agents preferred within the scope of the invention are ammonium carbamate, ammonia and/or ammonium(bi)carbonate, triethanolamine and monoethanolamine. It is desirable to adjust the pH-value between about 6.5 and 10.5, preferably about 7 to 9.5.

The viscosity best suited for the permanent waving compositions according to the invention proved to be in the range of about 500 to 10,000 mPa.s, preferably about 1,000 to about 5,000 mPa.s, measured at 20°C in a Brookfield viscosimeter (no. 5 spindle), whereas the viscosity suited for the straightening compositions is preferably higher in a range up to about 50,000 mPa.s, preferably up to 30,000 mPa.s measured at 20°C in a Brookfield viscosimeter (no. 5 spindle).

The viscosity is adjusted by addition of the appropriate amounts of thickening agents known per se, such as cellulose derivatives. Thickening may as well be realised by formulating a composition in form of an emulsion with the use of C₁₀-C₂₂-fatty alcohols, in admixture with long mono alkyl chain quaternary ammonium surfactants.

The intermediate composition of the inventive process of the present invention comprises ubiqinone which is preferably selected from the ones where n is a number between 6 and 10 and more preferably it is ubiqinone 50 where n is 10, also known as Coenzyme Q10.

Ubiquinones have been used in cosmetic compositions. Such compositions have been disclosed for example in EP 751 762 B1 for hair conditioning compositions in combination with retinols, in DE 199 26 167 A1 and DE 199 26 168 A1 for hair styling compositions, DE 199 26 170 A1 for cleansing preparations and in DE 199 26 156 A1 for hair conditioning compositions. Furthermore, in EP 1 232 741 A1 the use of ubiquinones in hair colouring compositions is disclosed.

In none of the patent documents mentioned above, a permanent shaping composition for hair comprising ubiquinone is neither disclosed nor mentioned.

Concentration of at least one ubiquinone of the above formula in the intermediate composition of the present invention is from 0.0001 to 1%, preferably from 0.0002 to 0.75%, more preferably from 0.0002 to 0.5% and most preferably from 0.0005 to 0.5% by weight, calculated to total composition.

Intermediate composition used in the inventive process of the present invention comprises further at least one salt, preferably in a concentration range from 0.5 to 15%, more preferably 1 to 12.5% and most preferably 2 to 12.5% by weight calculated to total composition.

In principal any water soluble salt is suitable for the purpose of the present invention. Preferably salt is an inorganic substance. In the preferred embodiment, salts are preferably selected from salts of mono or divalent cations with mono and divalent anions. Preferred cations are sodium, calcium, potassium and magnesium and anions are chloride and sulfate. Suitable ones are such as sodium chloride, sodium sulfate, magnesium sulfate, potassium chloride, potassium sulfate, magnesium chloride, calcium chloride, ammonium salts such as ammonium chloride and ammonium sulfate. Preferred salts are sodium chloride, sodium sulfate, magnesium sulfate, potassium chloride, potassium sulfate, magnesium chloride, and calcium chloride. More preferably the salts are sodium chloride, sodium sulfate, magnesium sulfate, potassium chloride, potassium sulfate and magnesium chloride. In particular, with magnesium sulfate and sodium chloride exceptionally good results were observed.

Intermediate treatment composition comprises at least one oxidizing agent at a concentration of 0.1 to 5%, preferably 0.2 to 5% more preferably 0.2 to 3% and most preferably 0.2 to 2% by weight calculated to total composition. Suitable oxidizing agents are such as hydrogen peroxide and sodium bromate. Most preferred is hydrogen peroxide.

The intermediate treatment composition has a pH between 2 and 7, preferably 2.5 and 6 and more preferably 3 and 5.

Further, according to a further preferred embodiment, intermediate treatment compositions comprise at least one cationic surfactant, quaternary ammonium surfactant and more preferably according to general formula 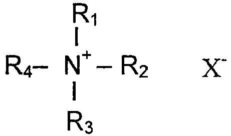 where R₁ is a saturated or unsaturated, branched or non-branched alkyl chain with 8-22 C atoms or

R₅CO NH (CH₂)ₙ

where R₅ is saturated or unsaturated, branched or non-branched alkyl chain with 7-21 C atoms and n has typical value of 0 - 4 or

R₆CO O (CH₂)ₙ

where R₆ is saturated or unsaturated, branched or non-branched alkyl chain with 7-21 C atoms and n has typical value of 0 - 4, and
R₂ is a hydrogen, saturated or unsaturated, branched or non-branched alkyl chain with 1-22 C atoms or

R₅CO NH (CH₂)ₙ

where R₅ is saturated or unsaturated, branched or non-branched alkyl chain with 7-21 C atoms and n has typical value of 0 - 4 or

R₆ CO O (CH₂)ₙ

where R₆ is saturated or unsaturated, branched or non-branched alkyl chain with 7-21 C atoms and n has typical value of 0 - 4,
and R₃ and R₄ are independent from each other H or lower alkyl chain with 1 to 4 carbon atoms, and X is chloride, bromide or methosulfate.

Concentration of at least one cationic surfactant is in the range from 0.05 % to 5 %, preferably 0.1 % to 2.5 %, more preferably 0.2 to 2% by weight, calculated to total of intermediate treatment composition.

Suitable long-chain quaternary ammonium compounds which can be used alone or in mixture are in particular cetyl trimethyl ammonium chloride, dimethyl dicetyl ammonium chloride, stearyl trimethyl ammonium chloride, behentrimonium chloride, cetyltrimethyl ammonium chloride, steartrimonium chloride, behentrimonium chloride, palmitoamidopropyl trimethyl ammonium chloride, stearamidopropyl trimethylammonium chloride, behenamidopropyl tri hydroxyethalmonium chloride, distearylamidopropyl dimethyl ammonium chloride, dicetylamidodihydroxyethyl ammonium chloride, palmitylpropyl trimethyl ammonium chloride, stearylpropyl trimethylammonium chloride, behenylpropyl tri hydroxyethalmonium chloride, distearylpropyl dimethyl ammonium chloride, dicetyldihydroxyethyl ammonium chloride, dioleoylethylhydroxyethylmonium methosulfate, and dicocoylethylhydroxyethylmonium methosulfate.

The third component used in the inventive process is a composition comprising at least one oxidizing agent. Suitable ones are such as hydrogen peroxide and sodium bromate. Most preferred is hydrogen peroxide. Oxidizing agent is comprised at a concentration of 0.05 to 15%, preferably 0.1 to 15% more preferably 0.25 to 12.5% and most preferably 0.5 to 10% by weight calculated to total composition. The oxidizing composition has a pH between 2 and 7, preferably 2.5 and 6 and more preferably 3 and 5.

Any of the compositions used in the inventive process of the present invention and the intermediate composition can comprise one or more of the following ingredients.

The compositions preferably comprise surfactants selected from anionic, nonionic and amphoteric ones. Cationic surfactants can as well be comprised in reducing and in oxidizing compositions. Their proportion ranges from about 0.05 % to about 10%, in particular from about 0.1 % to about 5 % by weight, calculated to total of each composition.

Suitable anionic surfactants are especially the known alkyl ether sulfates and carboxylic acids, in particular in form of their alkali salts, as well as protein fatty acid condensates.

Suitable nonionic surfactants, which are preferred within the scope of the invention, are in particular C₈-C₁₈-fatty alcohol polyglycol ethers, fatty acid polyglycol esters, fatty acid alkanolamides, amineoxides, and especially C₈-C₁₈-alkyl polyglucosides. Also possible is the incorporation of amphoteric surfactants, such as the known alkyl betaines, alkyl amido betaines, and alkyl amphoacetates.

In a further preferred embodiment of the present invention, the compositions comprise at least one cationic polymer. Basically suitable are all cationic polymers listed under the generic name "Polyquaternium" and "Quaternium" in the CTFA International Cosmetic Ingredient Dictionary. Examples are Polyquaternium 6, Polyquaternium 7, Polyquaternium 10, Polyquaternium 11, Polyquaternium 16, Polyquaternium 22 and Polyquaternium 28, Polyquaternium 39, Polyquaternium 87, Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-81, Quaternium-82, Quaternium-83 and Quaternium-84.

The cationic polymers also include the quaternized products of graft polymers from organopolysiloxanes and polyethyl oxazolines described in EP-A 524 612 and EP-A 640 643.

Concentration of one or more cationic polymers is in the range from 0.05 % to 5 %, preferably 0.1 % to 2.5 % by weight, calculated to total of each composition.

Compositions can comprise additionally at least one organic solvent. Suitable organic solvents are 2-methyl-1,3-propanediol, mono and dialcohols or the ethers thereof, in particular mono-C₁-C₃-alkyl ether, ethanol, n-propanol, isopropyl alcohol, 1-methoxypropanol, 1-ethoxypropanol and ethoxydiglycol, diols and their esters 1,3-and 1,4-butanediol, diethyleneglycol and the monomethyl and monoethyl ether thereof, dipropylene glycol and the monomethyl and monoethyl ether thereof, glycerol, hexanetriol, ethyl carbitol, benzyl alcohol, benzyloxy ethanol, propylene carbonate, N-alkyl pyrrolidone, and urea or their mixture preferably in an amount from about 0.1 % to 10 % by weight, calculated to the total of each composition. Composition can also comprise at least one amino acid. At least one amino acid is comprised at a concentration of 0.01 to 10%, preferably 0.05 to 7.5% and more preferably 0.1 to 5% and most preferably 0.25 to 5% by weight calculated to total of each composition.

Suitable amino acids are glycin, histidine, citrullin, asparagine, alanin, valin, leucin, isoleucin, pyrolin, tryptophane, phenylalanine, methionine, serine, tyrosine, threonine and gluatamine. Preferably, the amino acid is selected from glycin, histidine, citrullin, asparagine, alanin, valin, leucin, isoleucin, pyrolin, serine, tyrosine, threonine and gluatamine. More preferably, at least one amino acid is selected from glycin, histidine, asparagine, alanin, valin, leucin, pyrolin, serine, tyrosine and gluatamine, and most preferably at least one amino acid is selected from glycin, asparagine, alanin, valin, leucin, and serine.

Composition can comprise further ceramide type of compound with the general formula 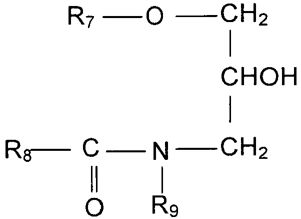 where R₇ and R₈ are independent from each other alkyl- or alkenyl group with 10 to 22 carbon atoms, R₉ is alkyl or hydroxyl alkyl with 1 to 4 carbon atoms group and n is a number between 1 to 6, preferably 2 or 3. Preferred compound according to the above chemical structure is cetyl-PG-hydroxyethylpalmitamide.

Further optional ingredient are sterols, especially the phytosterols as preferred hair restructuring agents. Especially preferred ones are of plant origin for example ergosterol, sitosterol, stigmasterol, fucosterol, brassicasterol, fungisterol, campesterol, zymosterol, ascosterol, cerevisterol, episterol, faecosterol, spinasterol. Among those phytosterols, the ones found in "Avocadin" which is the unsaponified fraction of the avocado oil is more preferred.

Optionally fatty acids of C₁₀ to C₂₂ may be incorporated into the compositions of the present invention at a concentration of preferably 0.01 to 2.5% by weight calculated to total of each composition.

In a further preferred embodiment of the present invention, intermediate treatment composition comprises at least one diamine compound. Preferred diamide compounds are according to the general structure 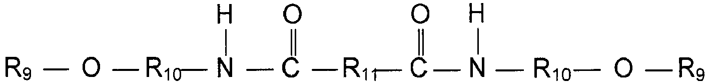 wherein R₉ is a linear or branched, saturated or unsaturated alkyl chain with 1 to 12 C atoms which may be substituted with hydroxy and/or alkoxy groups, preferably R₉ is linear or branched, saturated or unsaturated alkyl chain with 1 to 12 C atoms which may be substituted by 1 to 3 substituents selected from a hydroxy group and C1 to C6 alkoxy group, more preferably R₉ is a unsubstituted alkyl group with 1 to 12 C atoms, and alkyl group with 2 to 12 C atoms substituted by one or two hydroxyl groups, by one alkoxy group with 1 to 6 C atoms or by one hydroxyl and one alkoxy group with 2 to 6 C atoms, R₁₀ is linear or branched alkyl chain with 1 to 5 C atoms, preferably linear or branched alkyl chain with 2 to 5 C atoms and more preferably an alkyl chain with 2 to 3 C atoms, and R₁₁ linear or branched, saturated or unsaturated alkyl chain with 1 to 22 C atoms, preferably linear or branched, saturated or unsaturated alkyl chain with 11 to 22 C atoms.

Preferred individual diamide compounds are the ones according to the formula A to G.

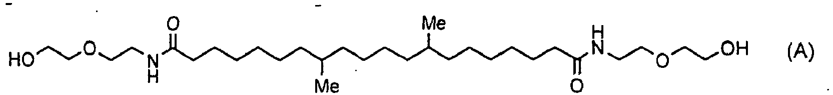

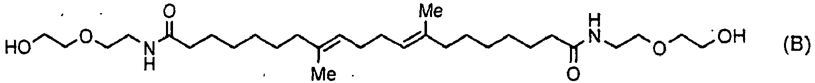

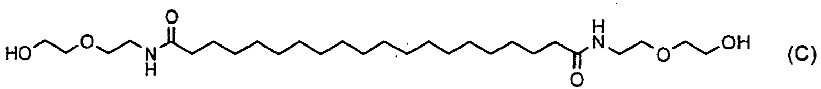

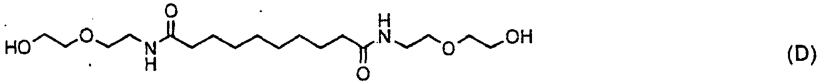

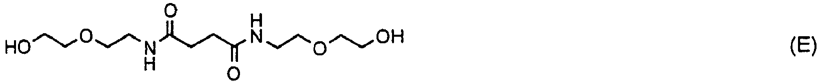

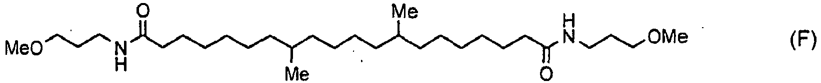

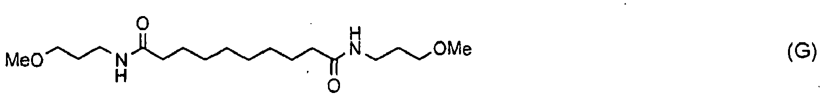

Particularly preferred diamide compound is the compound F which is bis (methoxypropylamido) isodocosane and commercially available from Kao Corporation - Japan.

Concentration of diamide compounds in the intermediate treatment compositions of the present invention is in the range of 0.001 to 5%, preferably 0.002 to 3% more preferably 0.005 to 2% and most preferably 0.01 to 1% by weight calculated to total of each composition.

Another preferred compound in the permanent shaping composition is silicone compounds and especially aminated silicones such as amodimethicone available from for example Dow Corning under the brand names Dow Corning 949 Emulsion and Dow Corning 2-8194 ME. Concentration of silicones, especially amodimethicone, is in the range of 0.05 to 2.5%, preferably 0.1 to 1% by weight calculated to total or each composition.

Additionally, one or more natural oil component may be incorporated into the compositions of the present invention. Suitable are such as olive oil, almond oil, avocado oil, wheatgerm oil, ricinus oil or their mixture. Concentration of these natural oil ingredients should be 0.01 to 2.5%, preferably 0.01.to 1%, more preferably 0.05 to 0.5% by weight, calculated to total each composition.

The compositions used according to the invention can naturally comprise all the substances customarily found in permanent shaping compositions, a list of which will not be given here, and are preferably present as solutions, gels with a higher or lower viscosity, emulsions or creams. They can be single-phase products or compositions packed into separate packaging which are united upon application, as they are disclosed, for example, in DE-C 43 04 828.

In order to avoid repetition, reference is here made to the state of the art as it is described, for example, in "Ullmann's Encyclopedia of Industrial Chemistry", Vol. A12 (1986), pages 588 to 591, and in particular to the monography of K. Schrader, "Grundlagen und Rezepturen der Kosmetika", 2nd. Ed. (1989, Hüthig Buchverlag) pages 823 to 840, as well as the article by D. Hollenberg et. al. in "Seifen-Ole-Fette-Wachse", 117 (1991), pages 81 to 87.

A straightening may also be carried out according to a process hair is washed and/or shampooed and reducing composition is applied onto hair and processed for 5 to 60 min, preferably 5 to 45 min and rinsed off with water and an intermediate composition comprising at least one ubiqinone of the formula 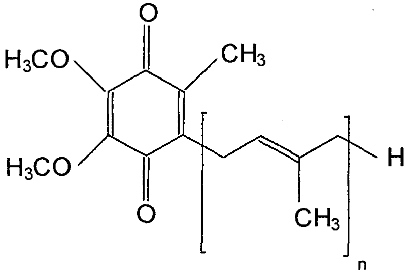 where n is a number between 1 and 10, and at least one salt and at least one oxidizing agent is applied onto hair and after rinsing off hair is dried and the dry hair physically straighten with hot iron at a temperature of 80 to 210°C and subsequently an oxidizing composition comprising at least one oxidizing agent, preferably hydrogen peroxide or sodium bromate at a concentration of 0.5 to 10% by weight calculated to total of oxidizing composition, is applied onto hair and left on the hair 1 to 20 min and rinsed off.

The compositions used in the inventive process of the present invention are preferably provided in a kit. Accordingly further subject of the present invention is a kit for permanent shaping keratin fibers preferably hair comprising a first composition comprising at least one reducing agent and a second composition comprising at least one ubiqinone of the formula 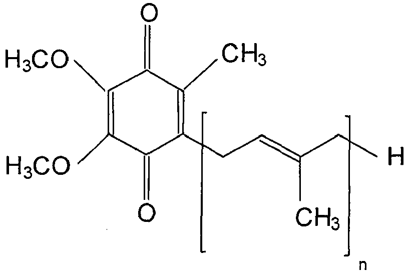 where n is a number between 1 and 10, and at least one salt and at least one oxidizing agent and a third composition comprising at least one oxidizing agent.

It has further been observed when other compositions used in the process also comprise at least one ubiqinone , the effects mentioned above are observed much stronger. Therefore, further object of the present invention is that a process for permanent shaping hair wherein a composition comprising at least one reducing agent and at least one ubiqinone of the above structure is applied onto hair and after processing of 1 to 30 min at a temperature of 20 to 45°C rinsed off from hair and an intermediate composition comprising at least one ubiqinone of the above formula, and at least one salt and at least one oxidizing agent is applied onto hair and after processing of 10 seconds to 10 min and without rinsing off a composition comprising at least one oxidizing agent and at least one ubiqinone of the above structure is applied and rinsed off from hair after a processing time of 1 to 30 min and at a temperature of 20 to 45°C.

The following examples are to illustrate the invention, but not to limit it.

### Example 1:

### Alkaline Permanent Wave for Normal Hair

| | % by weight |
|---|---|
| Ammonium thioglycolate (60%) | 21.3 |
| Ammonium hydrogen carbonate | 5.0 |
| 1,3- butylene gylcol | 3.0 |
| Amodimethicone | 0.2 |
| PEG-40-Hydrogenated castor oil | 0.7 |
| Polyquaternium-7 | 0.5 |
| Perfume | 0.4 |
| Ammonia, 25% | ad pH 8.3 |
| Water | ad 100.0 |

## Claims

1. Process for permanent shaping hair **characterized in that** a composition comprising at least one reducing agent is applied onto hair and after processing of 1 to 30 min at a temperature of 20 to 45°C rinsed off from hair and an intermediate composition comprising at least one ubiquinone of the formula 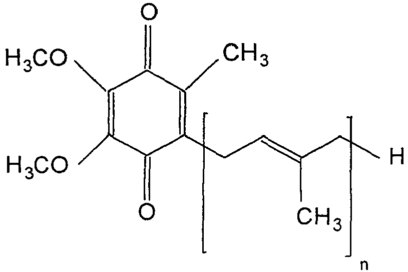 where n is a number between 1 and 10, and at least one salt and at least one oxidizing agent is applied onto hair and after processing of 10 seconds to 10 min and without rinsing off a composition comprising at least one oxidizing agent is applied and rinsed off from hair after a processing time of 1 to 30 min and at a temperature of 20 to 45°C.

2. Process according to claim 1 **characterized in that** the intermediate composition comprises ate least one cationic surfactant, preferably quaternary ammonium surfactant.

3. Process according to claims 1 and 2 **characterized in that** hair is washed with a shampoo and put on curlers prior to application of a composition comprising reducing agent.

4. Process according to claim 3 **characterized in that** the curlers were removed from hair right after application of the intermediate treatment, or right after application of the oxidizing agent or at the end of the processing time of oxidizing agent.

5. Process according to claims 1 to 4 **characterized in that** intermediate treatment comprises 0.0001 to 1% by weight, of Ubiquinone of the formula, calculated to total composition.

6. Process according to claims 1 to 5 **characterized in that** intermediate treatment comprises 0.1 to 15% by weight, of salt, calculated to total composition, preferably selected from salts of mono or divalent cations with mono and divalent anions and more preferably selected from sodium chloride, magnesium sulphate.

7. Process according to claim 6 **characterized in that** intermediate treatment comprises magnesium sulphate and/or sodium chloride as a salt.

8. Process according to claims 1 to 7 **characterized in that** intermediate treatment comprises 0.1 to 5% by weight, of an oxidizing agent, calculated to total composition, preferably hydrogen peroxide.

9. Process according to any of the preceding claims 1 to 8 **characterized in that** one or more compositions comprises at least one surfactant selected from anionic, nonionic, cationic and amphoteric ones at a concentration of 0.05 to 10% by weight, calculated to total of each composition.

10. Process according to any of the preceding claims 1 to 9 **characterized in that** one or more of the compositions comprises at least one cationic polymer at a concentration of 0.05 to 5% by weight, calculated to total of each composition and/or at least one organic solvent at a concentration of 0.1 to 10% by weight, calculated to total of each composition, and/or at least one silicone compound, preferably aminated silicone at a concentration of 0.05 to 2.5% by weight, calculated to total of each composition and/or at least one diamide compound.

11. Process according to any of the preceding claims 1 to 10 **characterized in that** reducing and oxidizing compositions used comprise as well at least one ubiquinone according to the formula of claim 1.

## Patentansprüche

1. Verfahren zum dauerhaften Formen von Haar, **dadurch gekennzeichnet, dass** eine Zusammensetzung, welche mindestens ein Reduktionsmittel aufweist, auf das Haar aufgebracht wird und nach einem Einwirken von 1 Minute bis 30 Minuten bei einer Temperatur von 20 °C bis 45 °C aus dem Haar ausgespült wird und eine Zwischenzusammensetzung, welche mindestens ein Ubichinon der Formel 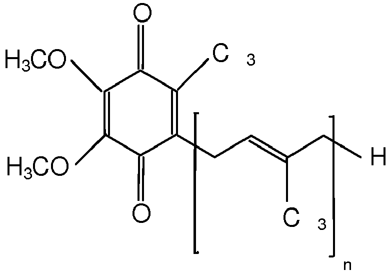 wobei n eine Zahl von 1 bis 10 ist, und mindestens ein Salz und mindestens ein Oxidationsmittel aufweist, auf das Haar aufgebracht wird und nach einem Einwirken von 10 Sekunden bis 10 Minuten und ohne Ausspülen eine Zusammensetzung, welche mindestens ein Oxidationsmittel aufweist, aufgebracht und nach einer Einwirkzeit von 1 Minute bis 30 Minuten und bei einer Temperatur von 20 °C bis 45 °C aus dem Haar ausgespült wird.

2. Verfahren nach Anspruch 1, **dadurch gekennzeichnet, dass** die Zwischenzusammensetzung wenigstens ein kationisches Tensid, vorzugsweise ein quartäres Ammonium-Tensid aufweist.

3. Verfahren nach Anspruch 1 und 2, **dadurch gekennzeichnet, dass** das Haar mit einem Shampoo gewaschen wird und vor dem Aufbringen einer Zusammensetzung, die ein Reduktionsmittel aufweist, auf Lockenwickler gezogen wird.

4. Verfahren nach Anspruch 3, **dadurch gekennzeichnet, dass** die Lockenwickler direkt nach dem Aufbringen der Zwischenbehandlung oder direkt nach dem Aufbringen des Oxidationsmittels oder am Ende der Einwirkzeit des Oxidationsmittels aus dem Haar entfernt werden.

5. Verfahren nach Anspruch 1 bis 4, **dadurch gekennzeichnet, dass** die Zwischenbehandlung 0,0001 Gewichts-% bis 1 Gewichts-% Ubichinon der Formel aufweist, bezogen auf die Gesamtzusammensetzung.

6. Verfahren nach Anspruch 1 bis 5, **dadurch gekennzeichnet, dass** die Zwischenbehandlung 0,1 Gewichts-% bis 15 Gewichts-% Salz aufweist, bezogen auf die Gesamtzusammensetzung, vorzugsweise ausgewählt aus Salzen ein- oder zweiwertiger Kationen mit ein- und zweiwertigen Anionen und insbesondere ausgewählt aus Natriumchlorid, Magnesiumsulfat.

7. Verfahren nach Anspruch 6, **dadurch gekennzeichnet, dass** die Zwischenbehandlung Magnesiumsulfat und/oder Natriumchlorid als ein Salz aufweist.

8. Verfahren nach Anspruch 1 bis 7, **dadurch gekennzeichnet, dass** die Zwischenbehandlung 0,1 Gewichts-% bis 5 Gewichts-% eines Oxidationsmittels aufweist, bezogen auf die Gesamtzusammensetzung, vorzugsweise Wasserstoffperoxid.

9. Verfahren nach einem der vorhergehenden Ansprüche 1 bis 8, **dadurch gekennzeichnet, dass** eine oder mehrere Zusammensetzungen mindestens ein Tensid, ausgewählt aus anionischen, nichtionischen, kationischen und amphoteren, in einer Konzentration von 0,05 Gewichts-% bis 10 Gewichts-% aufweisen, bezogen auf die Gesamtmenge der jeweiligen Zusammensetzung.

10. Verfahren nach einem der vorhergehenden Ansprüche 1 bis 9, **dadurch gekennzeichnet, dass** eine oder mehrere der Zusammensetzungen mindestens ein kationisches Polymer in einer Konzentration von 0,05 Gewichts-% bis 5 Gewichts-%, bezogen auf die Gesamtmenge der jeweiligen Zusammensetzung, und/oder mindestens ein organisches Lösungsmittel in einer Konzentration von 0,1 Gewichts-% bis 10 Gewichts-%, bezogen auf die Gesamtmenge der jeweiligen Zusammensetzung, und/oder mindestens eine Silikonverbindung, vorzugsweise aminiertes Silikon, in einer Konzentration von 0,05 Gewichts-% bis 2,5 Gewichts-%, bezogen auf die Gesamtmenge der jeweiligen Zusammensetzung, und/oder mindestens eine Diamidverbindung aufweisen.

11. Verfahren nach einem der vorhergehenden Ansprüche 1 bis 10, **dadurch gekennzeichnet, dass** reduzierende und oxidierende Zusammensetzungen, die verwendet werden, ebenso mindestens ein Ubichinon gemäß der Formel des Anspruchs 1 aufweisen.

## Revendications

1. Procédé pour une mise en forme permanente des cheveux **caractérisée en ce qu'**une composition comprenant au moins un agent réducteur est appliquée sur les cheveux, et après avoir laissé agir pendant 1 à 30 minutes à une température de 20 à 45 °C est éliminée par rinçage des cheveux et une composition intermédiaire, comprenant au moins une ubiquinone selon la formule 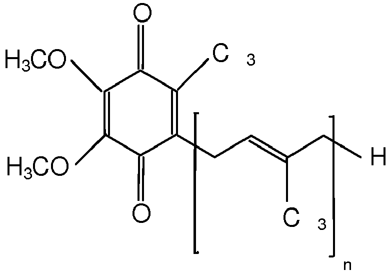 où n est un nombre entre 1 et 10, et au moins un sel et au moins un agent oxydant est appliquée sur le cheveux et après avoir laissé agir de 10 secondes à 10 minutes et sans rinçage, une composition comprenant au moins un agent oxydant est appliquée et éliminée des cheveux par rinçage après avoir laissé agir de 1 à 30 minutes et à une température de 20 à 45 °C.

2. Procédé selon la revendication 1, **caractérisé en ce que** la composition intermédiaire comprend au moins un tensio-actif cationique, de préférence un tensio-actif d'ammonium quaternaire.

3. Procédé selon les revendications 1 et 2, **caractérisé en ce que** les cheveux sont lavés avec un shampoing et enroulés sur des bigoudis avant l'application d'une composition comprenant l'agent réducteur.

4. Procédé selon la revendication 3, **caractérisé en ce que** les bigoudis ont été retirés des cheveux immédiatement après l'application du traitement intermédiaire, ou immédiatement après l'application de l'agent oxydant ou à la fin de la durée du traitement par l'agent oxydant.

5. Procédé selon les revendications 1 à 4, **caractérisé en ce que** le traitement intermédiaire comprend de 0,0001 à 1 % en poids d'Ubiquinone selon la formule, calculé par rapport à la composition totale.

6. Procédé selon les revendications 1 à 5, **caractérisé en ce que** le traitement intermédiaire comprend de 0,1 à 15 % en poids, calculé par rapport à la composition totale, de sel, de préférence choisi à partir des sels de cations mono ou divalents, avec des anions mono et divalents, et de manière plus préférable choisi à partir du chlorure de sodium, du sulfate de magnésium.

7. Procédé selon la revendication 6, **caractérisé en ce que** le traitement intermédiaire comprend du sulfate de magnésium et/ou du chlorure de sodium en tant que sel.

8. Procédé selon les revendications 1 à 7, **caractérisé en ce que** le traitement intermédiaire comprend de 0,1 à 5 % en poids, calculé par rapport à la composition totale, d'un agent oxydant, de préférence du peroxyde d'hydrogène.

9. Procédé selon l'une quelconque des revendications 1 à 8 précédentes, **caractérisé en ce qu'**une ou plusieurs compositions comprennent au moins un tensio-actif choisi à partir des tensio-actifs anioniques, non ioniques, cationiques et amphotères à une concentration de 0,05 à 10 % en poids, calculée par rapport à la totalité de chacune des compositions.

10. Procédé selon l'une quelconque des revendications 1 à 9 précédentes, **caractérisé en ce qu'**une ou plusieurs des compositions comprend au moins un polymère cationique à une concentration de 0,05 à 5 % en poids, calculée par rapport à la totalité de chacune des compositions, et/ou au moins un solvant organique à une concentration de 0,1 à 10 % en poids, calculée par rapport à la totalité de chacune des compositions, et/ou au moins un composé silicone, de préférence un silicone aminé à une concentration de 0,05 à 2,5 % en poids, calculée par rapport à la totalité de chacune des compositions, et/ou au moins un composé diamide.

11. Procédé selon l'une quelconque des revendications 1 à 10 précédentes, **caractérisé en ce que** les compositions réductrices et oxydantes utilisées comprennent également au moins une ubiquinone selon la formule de la revendication 1.
